# EUROPÄISCHE PATENTSCHRIFT

(11) **EP 2 453 930 B1**
(45) Veröffentlichungstag und Bekanntmachung des Hinweises auf die Patenterteilung: **18.03.2015**
(21) Anmeldenummer: 10724333.9
(22) Anmeldetag: 06.05.2010
(51) Int. Cl.: A61L 9/12

(54) **DUFTABGABESYSTEM MIT AKTIVIERUNGS- UND/ODER VERBRAUCHSANZEIGE**
FRAGRANCE-DISPENSING SYSTEM HAVING AN ACTIVATION AND/OR CONSUMPTION INDICATOR
SYSTÈME DIFFUSEUR DE PARFUM AVEC AFFICHAGE D'ACTIVATION ET/OU DE CONSOMMATION

(30) Priorität: 08.10.2009 DE 102009045481
(43) Veröffentlichungstag der Anmeldung: 23.05.2012
(73) Patentinhaber: Henkel AG & Co. KGaA, 40589 Düsseldorf (DE)
(72) Erfinder: MEIER, Frank, 40589 Düsseldorf (DE); SUNDER, Matthias, 40593 Düsseldorf (DE); FREEBORN, Matthew, 40217 Düsseldorf (DE)
(86) Internationale Anmeldenummer: PCT/EP2010/056136
(87) Internationale Veröffentlichungsnummer: WO 2011/042221

(56) Entgegenhaltungen:
- EP-A2- 0 309 173
- EP-A2- 0 462 605
- WO-A1-2004/006968
- WO-A2-2007/106547
- US-A1- 2008 251 599

## Beschreibung

Die Erfindung betrifft ein diffusionsgetriebenes Duftabgabesystem mit einer Aktivierungs- und/oder Verbrauchsanzeige.

### Stand der Technik

Diffusionsgetriebene Duftabgabesysteme sind hinlänglich aus dem Stand der Technik bekannt. Insbesondere sind Systeme basierend auf einem Docht und einem mit flüssiger, duftstoffhaltiger Zubereitung befülltem Behälter bekannt, bei denen der Docht in den Behälter eingeführt und die Zubereitung aufgrund der Kapillarkräfte des Dochtes entgegen der Schwerkraftrichtung zu den Oberflächen des Dochtes geführt wird, von wo aus der so transportierte Duftstoff an die Umgebung, üblicherweise über Verdampfung, abgegeben wird.

Nachteilig an derartigen Duftabgabesystemen ist es, dass vom Benutzer nicht erkannt werden kann, ob das System nach seiner erstmaligen Aktivierung korrekt funktioniert - also bestimmungsgemäß an die Umgebung abgibt - und/oder wann sich das System verbraucht hat.

### Aufgabe der Erfindung

Aufgabe der Erfindung ist es daher, ein Duftabgabesystem bereit zu stellen, dass auf einfache Weise einem Benutzer anzeigt, dass das Duftabgabesystem korrekt aktiviert wurde und/oder sich das Duftabgabesystem verbraucht hat.

Diese Aufgabe wird durch ein Duftabgabesystem mit den Merkmalen des Anspruchs 1 gelöst.

Das erfindungsgemäße Duftabgabesystem umfasst einen Docht und mindestens einen Diffusor, wobei die mit Umgebungsluft in Kontakt stehende Oberfläche des Diffusors größer ist als die mit der Umgebungsluft in Kontakt stehende Oberfläche des Dochtes. Somit bewirkt der Diffusor eine Vergrößerung der Oberfläche, über die Duftstoff an die Umgebung abgegeben wird.

Das erfindungsgemäße Duftabgabesystem umfasst des Weiteren einen Behälter zur Aufnahme des Dochts, sowie eine Duftstoffzubereitung, die in dem Behälter bevorratet ist, wobei die Duftstoffzubereitung in dem im Behälter aufgenommenen Zustand des Dochtes mittels Kapillarwirkung entgegen der Schwerkraftrichtung durch den Docht zum Diffusor transportiert wird. Der Diffusor ist bevorzugt an dem Behälter abgewandten Ende des insbesondere stiel- bzw. zylinderförmigen Dochtes angeordnet.

Innerhalb des kapillaren Strömungswegs der Duftstoffzubereitung durch den Docht und/oder Diffusor ist wenigstens ein von der Duftstoffzubereitung durchströmbares Depot mit mindestens einem von der Duftstoffzubereitung lösbaren Farbstoff vorgesehen.

Depot bedeutet im Sinne dieser Anmeldung ein definiertes Raumvolumen innerhalb des Duftabgabesystems innerhalb dessen wenigstens ein Farbstoff bevorratet ist.

Der im Depot befindliche Farbstoff wird durch die Duftstoffzubereitung beim Durchströmen des Depots, insbesondere aufgrund von Kapillarwirkung, gelöst, und färbt so längs des weiteren Diffusionswegs der Duftstoffzubereitung durch den Docht und/oder den Diffusor mit dem mittransportierten Farbstoff ein.

Es ist bevorzugt, dass der Docht und/oder der Diffusor aus einem synthetischen Material und/oder einem Fasermaterial pflanzlicher Herkunft, insbesondere einem cellulose-basiertem Material, gebildet sind/ist. Es ist selbstverständlich möglich, dass der Docht und der Diffusor aus gleichen oder unterschiedlichen Materialien gefertigt sind.

Vorteilhaft ist es ferner, dass der Diffusor in der Aufsicht eine im Wesentlichen kreisrunde Grundfläche aufweist, so dass der Abstand zwischen Docht, der insbesondere in der Mitte der kreisrunden Grundfläche mit dem Diffusor verbunden ist, und äußerem Rand des Diffusors im Wesentlichen konstant ist. Hierdurch ergeben sich über die Grundfläche hinweg im Wesentlichen gleiche Diffussionsweglängen und -zeiten.

Um eine höhere Abgabe von Duftstoff zu erwirken, dann es vorteilhaft sein, dass das Verhältnis aus der mit Umgebungsluft in Kontakt stehenden Oberfläche des Diffusors und der mit der Umgebungsluft in Kontakt stehenden Oberfläche des Dochtes zwischen 1.000:1 und 1,25:1, bevorzugt zwischen 125:1 und 2,5:1 liegt.

Bevorzugt ist der Diffusor aus einer Vielzahl von im Wesentlichen gleichartigen Lamellen gebildet ist, wobei ein Ende der Lamellen im bzw. am Docht fixiert ist. Es ist jedoch gemäß einer vorteilhaften Weiterentwicklung der Erfindung auch denkbar, dass der Diffusor aus wenigstens zwei Gruppen voneinander verschiedenen Lammellen gebildet ist.

Die Anzahl der Lamellen beträgt insbesondere mindestens 6, bevorzugt mindestens 50, insbesondere bevorzugt mindestens 100.

In einer weiteren besonders vorteilhaften Ausgestaltung der Erfindung weisen die Lammellen jeweils eine Oberfläche, bestehend aus Ober- und Unterseite einer Lammelle, von 0,5-100 cm², bevorzugt von 1-75 cm² insbesondere bevorzugt von 2-50 cm² auf.

Es ist ferner möglich, dass die Lamellen an der Dochtachse schraubig (azyklisch) oder wirtelig (zyklisch) angeordnet sind.

Bevorzugt beträgt der Winkel zwischen zwei benachbarten Lamellen zwischen 3° - 140°, bevorzugt zwischen 4°-137,5°.

Der aus den Lammellen gebildete Diffusor kann in einer weiteren Ausgestaltung der Erfindung wenigstens eine, bevorzugt zwei, insbesondere bevorzugt drei Symmetrieebenen aufweisen.

Der Fachmann kann aus dem Stand der Technik geeignete Farbstoffe für das Depot verwenden.

Ferner ist es insbesondere von Vorteil, dass das Depot im noch nicht im Behälter aufgenommenen Zustand von Außen nicht sichtbar im Docht und/oder Diffusor angeordnet ist.

Grundsätzlich ist es auch denkbar, dass im Docht und Diffusor Farbdepots mit jeweils voneinander verschiedenen Farbstoffen vorgesehen sind.

Der Farbstoff kann durch die dem Fachmann aus dem Stand der Technik bekannten Verfahren, beispielsweise durch Infusion (Einspritzen) in den Docht und/oder Diffusor eingebracht werden, so dass sich dort ein Depot an Farbstoff ausbildet.

Im Folgenden wird die Erfindung an Hand von einem exemplarischen Beispiel näher erläutert.

In einem ersten Ausführungsbeispiel besteht das Duftstoffabgabesystem aus einem Behälter für eine parfümölhaltige Duftstoffzubereitung, einem Docht, einem am Docht angeordneten Diffusor und einer parfümölhaltige Duftstoffzubereitung für die Verwendung mit dem Duftstoffabgabesystem.

Als Behälter wurde ein Glasgefäß mit einer ca. 1.5 cm weiten Öffnung verwendet werden. Es eignen sich jedoch prinzipiell auch Porzellan oder Keramikgefäße.

Als parfümölhaltige Duftstoffzubereitung können beliebige Zubereitungen z.B. von Parfümhäusern, wie Bell Flavors and Fragrances, Firmenich, Givaudan, International Fragrance&Flavors, Symrise oder Takasago verwendet werden, die für die Beduftung von Räumen in Dochtsystemen entwickelt wurden. Der Gehalt an Parfümöl im Lösungsmittel beträgt üblicherweise bei derartigen Duftstoffzubereitungen üblicherweise zwischen 2-10%.

Als Farbstoffe können beispielsweise die in Wasch- und Reinigungsmitteln üblichen Farbstoffe verwendet werden.

Als Lösungsmittel der duftstoffhaltigen Zubereitung eignet sich insbesondere ein Wasser/Ethanol Gemisch. Es können aber auch Isopropanol, Dipropyleneglycol, Diethylphthalate, Isopropyl-myristate, Benzyl-benzoate, 2-(2-ethoxy-ethoxy)-1-ethanol oder Ethyl Citrate alleine oder als Gemisch verwendet werden.

Als Docht- und/oder Diffusormaterial eignet sich insbesondere ein natürliches Pflanzenmaterial. Bei dem verwendeten Pflanzenmaterial handelt es sich um Aeschynomene aspera aus der Familie der Fabaceae.

Die Länge des Dochts beträgt bevorzugt ca. 8 cm bei einem Durchmesser von ca. 1 cm. Der Durchmesser des Dochtes ist so gewählt, dass er gut in die Öffnung des die duftstoffhaltige Zubereitung bevorratenden Behälters eingeführt werden kann und der Diffusor einige Zentimeter aus der Öffnung hinausragt. Der Behälter und der Docht sowie der Diffusor sind so konfiguriert und aufeinander abgestimmt, dass der Behälter mit dem eingeführten Docht und Diffusor standsicher ist. Der Durchmesser des Diffusors liegt bei ca. 5 cm.

Die Farbstoffe können beispielsweise ausgewählt sein aus nachfolgender Liste, die die Farbstoffe im C.I. (Color Index) mit ihrem generischen Namen bezeichnet:
Solvent Red 135, Acid red 18, Food red 7, Azo complex, Pigment Red 112, Pigment violet 23, Basic violet, Acid red 52, C.I. Food Yellow 3, Food red 9, Pigment red 112, FD&C yellow 5, Aluminium lake, Pigment yellow 1, Sunset yellow, Acid Yellow 5, Basic Yellow 28,Yellow 97, DC yellow 8, Acid green 25, Green 7, Patent Blue AE E133, Direct blue 86, Acid Blue 182, Acid blue 9, Acid blue 3, Basic blue 3, Acid blue 225, Blue 15:1, Blue 15:3, Acid blue 80, Acid blue 7, Acid blue 9, FD & C blue No. 1, Food Blue 2, VAT Red 1, Acid red 27, Solvent yellow 16, Pigment Yellow 1, Acid Yellow 9, Pigment green 7, Pigment blue 15:1, Pigment blue 15, Acid blue 9,
Acid red 52, Basic Violet 10, Basic Violet 10, Basic violet 10, Basic violet 10, Reactive Red 24:1, Acid yellow 232, Acid red 274, Acid brown 413, Acid red 414, Acid red 52, Acid red 18, Food red 7, Acid red 52, Basic violet 10, FD&C No 40, Food Red 17, Acid red 1, Food red 10, Solvent yellow 179, Disperse yellow 201, Acid yellow 36, Pigment yellow 147, Acid yellow 17, Acid orange 7, Yellow 81, Reactive yellow 161, Food yellow 3, Acid yellow 218, Solvent yellow 174, Acid yellow 3, Reactive yellow 25, Reactive yellow 25, Food yellow 13, Solvent yellow 93,
Solvent Orange, Pigment green 7, Solvent Green 3, Solvent Green 7, Pigment green 7, Pigment Green 7, Pigment Green 7, Acid green 25, Food Yellow 4, Acid Yellow 23, Direct blue 86, FD&C Blue No.1, Solvent blue 35, Acid blue 9, Acid Blue 104, Pigment blue 15:1, Acid blue 182, Solvent Blue 35, Acid blue 182, Acid blue 9, Blue 35, Reactive blue 197, Pigment blu 29, Pigment Blue 15:1, Acid blue 9, Food Blue 2, Acid blue 9.

Der Farbstoff ist den Innerem Lagen des Dochtes zugesetzt, beispielsweise durch Imprägnierung dieser Lagen mit dem Farbstoff oder durch Einspritzen des Farbstoffs in diese Lagen. Die äußeren Lagen sind bevorzugt nicht mit dem Farbstoff der inneren Lagen versehen, so dass ein Benutzer die Farbstoff enthaltenen inneren Lagen des Dochtes nicht sieht, bzw. nicht mit Ihnen bei der Verwendung des Dochtes in Berührung kommen kann.

## Patentansprüche

1. Duftabgabesystem umfassend
- einen Docht
- mindestens einen Diffusor,
o wobei die mit Umgebungsluft in Kontakt stehende Oberfläche des Diffusors größer ist als die mit der Umgebungsluft In Kontakt stehenden Oberfläche des Dochtes und
- einen Behälter zur Aufnahme des Dochts
- eine Duftstoffzubereitung, die in dem Behälter bevorratet ist
- wobei die Duftstoffzubereitung in dem Im Behälter aufgenommenen Zustand des Dochtes mittels Kapillarwirkung entgegen der Schwerkraftrichtung durch den Docht zum Diffusor transportiert wird,
**dadurch gekennzeichnet, dass**
innerhalb des kapillaren Strömungswegs der Duftstoffzubereitung durch den Docht und/oder Diffusor wenigstens ein von der Duftstoffzubereitung durchströmbares Depot mit mindestens einem von der Duftstoffzubereitung-lösbaren Farbstoff vorhanden ist

2. Duftabgabesystem nach einem der vorherigen Ansprüche, **dadurch gekennzeichnet, dass** das Depot im noch nicht im Behälter aufgenommenen Zustand von Außen nicht sichtbar im Docht und/oder Diffusor angeordnet ist.

3. Duftabgabesystem nach einem der vorherigen Ansprüche, **dadurch gekennzeichnet, dass** der Docht und/oder der Diffusor aus einem synthetischen Material und/oder einem Fasermaterial pflanzlicher Herkunft, insbesondere einem cellulose-basiertem Material, gebildet sind/ist.

4. Duftabgabesystem nach einem der vorherigen Ansprüche, **dadurch gekennzeichnet, dass** der Diffusor in der Aufsicht eine kreisrunde Grundfläche aufweist

5. Duftabgabesystem nach einem der vorherigen Ansprüche, **dadurch gekennzeichnet, dass** das Verhältnis aus der mit Umgebungsluft in Kontakt stehenden Oberfläche des Diffusors und der mit der Umgebungsluft in Kontakt stehenden Oberfläche des Dochtes zwischen 1.000:1 und 1,25:1, bevorzugt zwischen 125:1 und 2,5:1 liegt

6. Duftabgabesystem nach einem der vorherigen Ansprüche, **dadurch gekennzeichnet, dass** der Diffusor aus einer Vielzahl von gleichartigen Lamellen gebildet ist, wobei ein Ende der Lamellen im bzw. am Docht fixiert ist.

7. Duftabgabesystem nach einem der vorherigen Ansprüche, **dadurch gekennzeichnet, dass** der Diffusor aus wenigstens zwei Gruppen voneinander verschiedenen Lammellen gebildet ist

8. Duftabgabesystem nach Anspruch 6 oder 7, **dadurch gekennzeichnet**, dase die Anzahl der Lamellen mindestens 6, bevorzugt mindestens 60, insbesondere bevorzugt mindestens 100 beträgt.

9. Duftabgabesystem nach Ansprüchen 6-8, **dadurch gekennzeichnet, dass** die Lammellen jeweils eine Oberfläche, bestehend aus Ober- und Unterseite einer Lammelle, von 0,5-100 cm², bevorzugt von 1-75 cm² insbesondere bevorzugt von 2-50 cm² aufweisen.

10. Duftabgabesystem nach Ansprüchen 6-9, **dadurch gekennzeichnet, dass** die Lamellen an der Dochtachse schraubig oder wirtelig angeordnet sind.

11. Duftabgabesystem nach Ansprüchen 6-10, **dadurch gekennzeichnet, dass** der Winkel zwischen zwei benachbarten Lamellen zwischen 3° -140°, bevorzugt zwischen 4°-137,5° liegt.

12. Duftabgabesystem nach Ansprüchen 6-11, **dadurch gekennzeichnet, dass** der aus Lammellen gebildete Diffusor wenigstens eine, bevorzugt zwei, insbesondere bevorzugt drei Symmetrieebenen aufweist

13. Duflabgabesystem nach einem der vorherigen Ansprüche, **dadurch gekennzeichnet, dass** am Docht und Diffusor Farbdepots mit Jeweils voneinander verschiedenen Farbstoffen vorgesehen sind.

## Claims

1. A scent delivery system comprising
- a wick,
- at least one diffuser,
• wherein that surface of the diffuser which is in contact with ambient air is larger than that surface of the wick which is in contact with ambient air, and
- a container for receiving the wick,
- a scent preparation that is stored in the container,
- wherein when the wick is in the state of being received in the container, the scent preparation is transported by capillary action, against the force of gravity, through the wick to the diffuser, **characterized in that**
within the capillary flow path of the scent preparation through the wick and/or diffuser, at least one reservoir through which the scent preparation can flow, having at least one dye dissolvable by the scent preparation, is present.

2. The scent delivery system according to one of the preceding claims, **characterized in that** in the state of not yet being received in the container, the reservoir is arranged in the wick and/or diffuser invisibly from the outside.

3. The scent delivery system according to one of the preceding claims, **characterized in that** the wick and/or the diffusor is/are constituted from a synthetic material and/or from a fibre material of vegetable origin, in particular a cellulose-based material.

4. The scent delivery system according to one of the preceding claims, **characterized in that** the diffuser has a circular base area in plan view.

5. The scent delivery system according to one of the preceding claims, **characterized in that** the ratio of that surface of the diffuser which is in contact with ambient air to that surface of the wick which is in contact with ambient air is between 1000:1 and 1.25:1, preferably between 125:1 and 2.5:1.

6. The scent delivery system according to one of the preceding claims, **characterized in that** the diffuser is constituted from a plurality of lamellae of the same type, wherein one end of the lamellae is secured in or on the wick.

7. The scent delivery system according to one of the preceding claims, **characterized in that** the diffuser is constituted from at least two groups of lamellae that differ from one another.

8. The scent delivery system according to Claim 6 or 7, **characterized in that** the number of lamellae is at least 6, preferably at least 50, particularly preferably at least 100.

9. The scent delivery system according to Claims 6 to 8, **characterized in that** the lamellae each have a surface area, made up of the upper and lower side of a lamella, from 0.5 to 100 cm², preferably from 1 to 75 cm², particularly preferably from 2 to 50 cm².

10. The scent delivery system according to Claims 6 to 9, **characterized in that** the lamellae are arranged in helical or whorled fashion on the wick axis.

11. The scent delivery system according to Claims 6 to 10, **characterized in that** the angle between two adjacent lamellae is between 3° and 140°, preferably between 4° and 137.5°.

12. The scent delivery system according to Claims 6 to 11, **characterized in that** the diffuser constituted from lamellae has at least one, preferably two, particularly preferably three planes of symmetry.

13. The scent delivery system according to one of the preceding claims, **characterized in that** colour reservoirs each having dyes different from one another are provided on the wick and diffuser.

## Revendications

1. Système diffuseur de parfum, comprenant
- une mèche
- au moins un diffuseur,
- la surface du diffuseur en contact avec l'air ambiant étant supérieure à la surface de la mèche en contact avec l'air ambiant et
- un récipient pour recevoir la mèche
- une préparation parfumée qui est stockée dans le récipient,
- la préparation parfumée étant transportée, lorsque la mèche est dans l'état incorporé dans le récipient, par effet capillaire contre le sens de la gravité au travers de la mèche vers le diffuseur,
**caractérisé en ce qu'**au moins un dépôt, présentant au moins un colorant soluble dans la préparation parfumée, au travers duquel peut s'écouler la préparation parfumée, se trouve dans la voie d'écoulement capillaire de la préparation parfumée au travers de la mèche et/ou du diffuseur.

2. Système diffuseur de parfum selon l'une quelconque des revendications précédentes, **caractérisé en ce que** le dépôt, lorsqu'il est dans l'état non encore incorporé dans le récipient, est disposé dans la mèche et/ou le diffuseur de manière à ne pas être visible de l'extérieur.

3. Système diffuseur de parfum selon l'une quelconque des revendications précédentes, **caractérisé en ce que** la mèche et/ou le diffuseur est/sont formé(e)(s) à partir d'un matériau synthétique et/ou d'un matériau fibreux d'origine végétale, en particulier d'un matériau à base de cellulose.

4. Système diffuseur de parfum selon l'une quelconque des revendications précédentes, **caractérisé en ce que** le diffuseur présente une surface de base circulaire en vue du dessus.

5. Système diffuseur de parfum selon l'une quelconque des revendications précédentes, **caractérisé en ce que** le rapport de la surface du diffuseur en contact avec l'air ambiant à la surface de la mèche en contact avec l'air ambiant se situe entre 1000:1 et 1,25:1, de préférence entre 125:1 et 2,5:1.

6. Système diffuseur de parfum selon l'une quelconque des revendications précédentes, **caractérisé en ce que** le diffuseur est formé d'une multitude de lamelles de même type, une extrémité des lamelles étant fixée dans ou sur la mèche.

7. Système diffuseur de parfum selon l'une quelconque des revendications précédentes, **caractérisé en ce que** le diffuseur est formé à partir d'au moins deux groupes de lamelles différentes les unes des autres.

8. Système diffuseur de parfum selon la revendication 6 ou 7, **caractérisé en ce que** le nombre de lamelles est d'au moins 6, de préférence d'au moins 50, en particulier de préférence d'au moins 100.

9. Système diffuseur de parfum selon les revendications 6-8, **caractérisé en ce que** les lamelles présentent à chaque fois une surface, constituée par la face supérieure et la face inférieure d'une lamelle, de 0,5-100 cm², de préférence de 1-75 cm², en particulier de préférence de 2-50 cm².

10. Système diffuseur de parfum selon les revendications 6-9, **caractérisé en ce que** les lamelles sont disposées en hélice ou en verticille sur l'axe de la mèche.

11. Système diffuseur de parfum selon les revendications 6-10, **caractérisé en ce que** l'angle entre deux lamelles adjacentes est situé entre 3°-140°, de préférence entre 4°-137,5°

12. Système diffuseur de parfum selon les revendications 6-11, **caractérisé en ce que** le diffuseur formé par les lamelles présente au moins un, de préférence deux, en particulier de préférence trois plans de symétrie.

13. Système diffuseur de parfum selon l'une quelconque des revendications précédentes, **caractérisé en ce que** des dépôts colorés présentant à chaque fois des colorants différents les uns des autres sont prévus sur la mèche et le diffuseur.
